(19) Europäisches Patentamt
European Patent Office
Office européen des brevets

(11) Veröffentlichungsnummer: **0 004 611**
**A1**

(12) **EUROPÄISCHE PATENTANMELDUNG**

(21) Anmeldenummer: **79100881.6**

(22) Anmeldetag: **23.03.79**

(51) Int. Cl.$^2$: **C 07 C 102/00**
C 07 C 143/675, C 07 D 235/26

(30) Priorität: **31.03.78 CH 3479/78**

(43) Veröffentlichungstag der Anmeldung:
**17.10.79 Patentblatt 79/21**

(84) Benannte Vertragsstaaten:
**CH DE FR GB IT**

(71) Anmelder: **CIBA-GEIGY AG**
**Patentabteilung Postfach**
**CH-4002 Basel(CH)**

(72) Erfinder: **Hegner, Paul, Dr.**
**General Guisan-Strasse 14**
**CH-4054 Basel(CH)**

(54) **Verfahren zur Herstellung von Acetoacetylarylamiden.**

(57) Verfahren zur Herstellung von Acetoacetylarylamiden durch Anlagerung von Diketen an primäre carbocyclische oder heterocyclische aromatische Amine, wobei man das Diketen in die heterogene Suspension des Amins in einem Lösungsmittel oder Lösungsmittelgemisch bestehend aus gesättigten aliphatischen und/oder cycloaliphatischen Kohlenwasserstoffen mit 5 bis 18 C-Atomen zudosiert. Dieses neue Verfahren gestattet es ausserordentlich reine Produkte bei annähernd quantitativer Ausbeute zu erhalten, unter ökologisch unproblematischen Bedingungen.

EP 0 004 611 A1

Croydon Printing Company Ltd.

0004611

3-11658/+

CIBA-GEIGY AG, BASEL

# Verfahren zur Herstellung von Acetoacetylarylamiden

Die vorliegende Erfindung betrifft ein Verfahren zur Herstellung von Acetoacetylarylamiden durch Anlagerung von Diketen an primäre aromatische Amine bei Verwendung aliphatischer Kohlenwasserstoffe als Lösungsmittel.

Die Analgerung von Diketen an primäre aromatische Amine zur Herstellung von Acetoacetylarylamiden ist bekannt. In J.Am.Chem.Soc. 68, 644-647 (1946) beschreiben C.E. Kaslow und N.B. Sommer beispielsweise ein Verfahren, bei dem 2,5-Dimethoxyanilin mit Diketen zu 2,5-Dimethoxyacetoacetanilid umgesetzt wird, bei dem als Lösungsmittel Benzol verwendet wird. Es wird dabei wohl eine Ausbeute von 94% d.Th. aber kein Reingehalt des Produktes angegeben. Aus dem erwähnten Schmelzpunkt von 63-66°C ist allerdings zu entnehmen, dass der Reingehalt verhältnismässig tief liegt wenn man gedenkt, dass reines 2,5-Dimethoxy-acetoacetanilid über 70°C schmilzt. Die Tatsache, dass die hergestellten Produkte in Benzol noch gut löslich sind, erschwert ausserdem auch deren Isolierung.

In der DE-OS 2 519 036 wird ein homogenes, wässriges Verfahren beschrieben, bei dem vor der Diketenzugabe das Ausgangsamin durch Erwärmen und/oder durch Zugabe einer grösseren Menge Essigsäure in Lösung gebracht wird. Das Reaktionsgemisch enthält bis zu 60 Gew.-% Essigsäure und das Diketen muss möglichst rasch zudosiert werden. Man erhält sehr reine Produkte in guter Ausbeute, im allgemeinen über 90% d.Th.

Ein weiteres Verfahren in Wasser oder verdünnte Essigsäure ist speziell für 2,5-Dimethoxy-4-chlor-anilin in der DE-AS 2 518 984 beschrieben. Dabei wird das Amin in Suspension mit einem relativ grossen Ueberschuss von 1,5 Mol Diketen pro Mol Amin umgesetzt. Das Diketen wird auf einmal zugegeben. Es werden wohl gute Ausbeuten von 94-96% d.Th. aber kein Reingehalt der Produkte angegeben.

Die DE-AS 2 518 922 beschreibt ein Verfahren zur Herstellung von 5-Acetoacetylaminobenzimidazolon-(2) durch Umsetzung von Diketen mit 5-Aminobenzimidazolon-(2) in Form eines Salzes einer Säure mit pKa-Wert von 1 bis 7 in wässriger Lösung. Diese Arbeitsweise ist jedoch verfahrenstechnisch unbefriedigend. Infolge einer Konkurrenzreaktion zwischen Diketen und heissem Wasser kommt es zu einer heftigen Kohlendioxidentwicklung, die mit einem starken Schäumen des Reaktionsgemisches und Aufrahmen des Reaktionsproduktes verbunden ist. Die Reinigung der Abgase erfordert zudem einen hohen technischen Aufwand. Auch die Ausbeuten liegen relativ tief, im allgemeinen unter 90% d.Th. Diese Nachteile können wohl durch ein in der DE-OS 2 612 391 beschriebenes kontinuierliches Verfahren vermieden werden, welches allerdings geringere Ausbeuten liefert.

Alle beschriebenen Verfahren auf der Basis wässriger Systeme sind ökologisch problematisch, insbesondere bezüglich der neueren Bestimmungen über Abwasserreinigung.

Es wurde nun gefunden, dass überraschenderweise die Diketenisierung von primären carbocyclischen oder heterocyclischen aromatischen Aminen auch ohne die obengenannten Nachteile zu ausserordentlich reinen Produkten bei annähernd quantitativer Ausbeute führt, wenn man das Diketen in die heterogene Suspension des Amins in einem Lösungsmittel oder Lösungsmittelgemisch, bestehend aus gesättigten aliphatischen und/oder cycloaliphatischen Kohlenwasserstoffen mit 5 bis 18 C-Atomen zudosiert.

Dies ist umso erstaunlicher, da sowohl die Ausgangsprodukte als auch die Endprodukte in solchen Lösungsmitteln nur spurenweise löslich sind.

Im erfindungsgemäss einzusetzenden Lösungsmittel können bis zu höchstens 20 Gew.-% aromatische Kohlenwasserstoffe enthalten sein, bezogen auf die gesamte Lösungsmittelmenge.

Für das erfindungsgemässe Verfahren besonders interessante Lösungsmittel sind geradkettige oder verzweigte Paraffine mit 5 bis 18 C-Atomen, wie beispielsweise n-Pentan, n-Hexan, n-Heptan, n-Oktan, n-Nonan, n-Decan, n-Undecan, n-Dodecan, n-Tridecan, n-Tetradecan, n-Pentadecan, n-Hexadecan, n-Heptadecan, n-Octadecan, Dimethyl- und Trimethylbutan, Methyl-, Dimethyl- und Trimethylpentan, Methyl-, Dimethyl- und Polymethyl-Hexan, -Heptan, -Oktan, -Decan, -Dodecan, -Pentadecan, Aethyl-, Diäthyl- und Polyäthyl-Hexan, -Heptan, -Oktan, -Nonan, -Decan, -Undecan, -Tridecan, Propyl-, Dipropyl-Pentan, -Hexan oder -Oktan.

Als Lösungsmittel ebenfalls von besonderem Interesse für das erfindungsgemässe Verfahren sind Cycloparaffine mit 5 bis 18 C-Atomen, wie beispielsweise Cyclopentan, Methyl- und Aethylcyclopentan, Cyclohexan, Methyl- und Dimethylcyclohexan, Aethyl- und Diäthylcyclohexan, Cycloheptan, Methyl-, Dimethyl-, Trimethyl-, Aethyl-, Diäthyl-, Triäthylcycloheptan, Cyclooctan, Cyclononan, Cyclodecan, Cyclododecan und ihre Methyl-, Dimethyl-, Trimethyl-, Aethyl-, Diäthyl- und Triäthylderivate, Cyclohexadecan und Cyclooctadecan.

Besonders geeignet für das erfindungsgemässe Verfahren sind auch Lösungsmittelgemische aus geradkettigen, verzweigten und/oder cyclischen Paraffinen wie sie oben beschrieben sind.

Für das erfindungsgemässe Verfahren besonders bevorzugte Lösungsmittel sind beispielsweise die handelsüblichen Produkte wie die Siedegrenzenbenzine von 100/125°C und 110/140°C oder die SHELL-Produkte Shellsol K, Shellsol T oder Shellsol TD, sowie Ligroin, Hexan oder Solvent 30 (Schweizerische Sprengstoff-Fabrik AG, Dottikon).

Das erfindungsgemässe Verfahren eignet sich sehr gut zur Diketenisierung von Aminobenzolen, insbesondere solche der Formel I

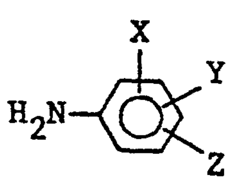

(I)

worin X und Y jeweils unabhängig voneinander Wasserstoff, Halogen, Alkyl oder Alkoxy mit 1-4 C-Atomen, Benzyl und Z Wasserstoff, Chlor, Methyl, Trifluormethyl, Nitro, Cyano, Alkanoylamino mit 1-4 C-Atomen oder eine Gruppe der Formeln

$-CONH_2$, 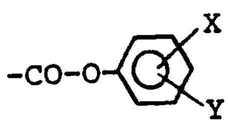 , 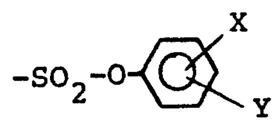 , 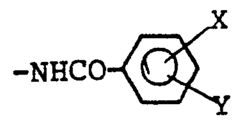

oder -COOR

bedeuten, worin X und Y die oben angegebene Bedeutung haben und R Alkyl mit 1-4 C-Atomen ist.

Ebenso geeignet ist das erfindungsgemässe Verfahren für die Diketenisierung von Diaminobenzolen, insbesondere solche der Formel II

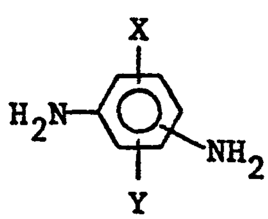

(II)

worin X und Y die oben angegebene Bedeutung haben.

Sehr gut eignet sich das erfindungsgemässe Verfahren auch zur Diketenisierung von Diaminodiphenylen, insbesondere solche der Formel III

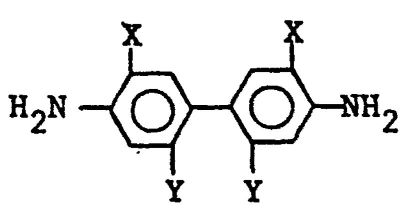

(III)

worin X und Y die oben angegebene Bedeutung haben.

Das erfindungsgemässe Verfahren ist auch zur Diketenisierung von heterocyclischen aromatischen Aminen zu empfehlen, insbesondere jene der Formel IV

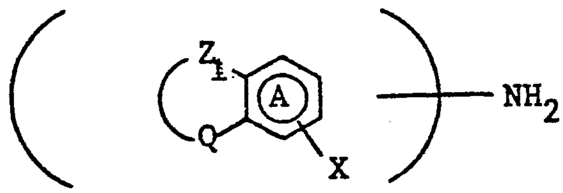

(IV)

worin X die oben angegebene Bedeutung hat, Q eine Gruppe der Formeln -CONH- oder -NHCO- und $Z_1$ ein Sauerstoffatom oder eine Gruppe der Formeln $-NR^1-$, -NHCO-, CONH-, $-\underset{R^1}{C}=CH-$. -CH=N- oder

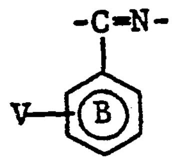

darstellt, wobei $R^1$ Wasserstoff, Alkyl mit 1-4 C-Atomen oder gegebenenfalls durch Halogen, Alkyl oder Alkoxy mit 1-4 C-Atomen substituiertes Phenyl und V Wasserstoff, Halogen, Alkyl oder Alkoxy mit 1-4 C-Atomen bedeuten und die Aminogruppe an die Phenylreste A oder B gebunden sein kann.

Besonders bevorzugte heterocyclische Amine, die nach dem erfindungsgemässen Verfahren diketenisiert werden können sind diejenigen der Formel V

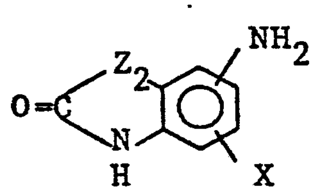

(V)

worin X die oben angegebene Bedeutung hat und $Z_2$ ein Sauerstoffatom oder die Gruppe -NH- bedeutet.

Stellen etwaige Substituenten Halogen dar, so handelt es sich insbesondere um Chlor oder Brom, vorzugsweise um Chlor.

Bedeuten etwaige Substituenten Alkyl, so stellen sie beispielsweise Methyl, Aethyl, n-Propyl, Isopropyl, n-Butyl, sek.-Butyl oder tert.-Butyl dar, vorzugsweise jedoch Methyl oder Aethyl.

Stellen etwaige Substituenten Alkoxygruppen dar, so bedeuten sie z.B. Methoxy, Aethoxy, n-Propoxy, Isopropoxy, n-Butoxy oder tert.-Butoxy, insbesondere jedoch Methoxy oder Aethoxy.

Bedeutet Z Alkanoylamino, so stellt es Acetylamino, Propionylamino, n-Butyrylamino oder Isobutyrylamino dar, vorzugsweise jedoch Acetylamino.

Die Diketenisierung wird vorzugsweise so durchgeführt, dass das Amin mit nur so wenig aliphatischem Lösungsmittel angeschlämmt wird, dass der Ansatz gut rührbar ist. Die somit erhaltene Suspension wird anschliessend auf 0-5°C abgekühlt und bei kräftigem Rühren mit 1,05 bis höchstens 1,3 Mol Diketen pro Mol Monoamin (oder pro 1/2 Mol Diamin) möglichst langsam versetzt, damit die Temperatur während dem Zulauf vorzugsweise 15°C nicht überschreitet. Nach Beendigung der Diketen-Zugabe lässt man so lange bei niedriger Temperatur reagieren, bis im Dünnschichtchromatogramm kein Ausgangsamin mehr nachweisbar ist.

Die Temperatur ist an und für sich für das erfindungsgemässe Verfahren nicht kritisch. Besonders reine Produkte erhält man jedoch bei Temperaturen zwischen -30 bis +30°C und insbesondere zwischen -5 und +15°C.

Zur Beschleunigung der Endreaktion kann nach dem Diketenzulauf das Gemisch bis auf höchstens 100°C aufgeheizt werden. Ein Zusatz von kleinen Mengen Wasser oder Essigsäure ist manchmal sehr angezeigt. Daher ergibt sich sogar

die Möglichkeit wasserfeuchte Amine gegebenenfalls tel quel einzusetzen.

Die nach dem Filtrieren des entstandenen Produktes erhaltene Mutterlauge kann in den meisten Fällen tel quel wieder eingesetzt werden, wobei der Diketen-Ueberschuss mit recycliert wird.

Das erfindungsgemässe Verfahren weist besonders wichtige Vorteile bezüglich Wirtschaftlichkeit und Oekologie auf:

- Ausgezeichnete Ausbeute, annähernd quantitativ
- Geringer Diketen-Ueberschuss
- Hohe Raumzeit-Ausbeute wegen der hohen Arbeitskonzentration in Suspension
- Verwendung von billigen, leicht regenerierbaren Lösungsmitteln oder Lösungsmittelgemischen
- Mögliche Recyclisierung der Reaktionsmutterlauge mit dem noch vorhandenen Diketen-Ueberschuss
- Mögliche direkte Weiterverwendung der Reaktionssuspension für die Durchführung der nächsten Synthese-Stufe
- Keine Abwasserbelastung
- Praktisch keine Rückstände, infolge der hohen Umsätze.

Die nach dem erfindungsgemässen Verfahren hergestellten Acetoacetylarylamide werden insbesondere als Kupplungskomponenten bei der Herstellung von Azofarbstoffen, bevorzugt Azopigmenten eingesetzt.

Die nachfolgenden Beispiele dienen der näheren Erläuterung der vorliegenden Erfindung.

**Beispiel 1**

a) 225,3 g 4-Amino-acetanilid werden in 1500 ml Shellsol K von 100/125°C (Aromatengehalt 0,5 %) angerührt und mit einem Eisbad auf 5°C gekühlt. Darauf lässt man innert 2-3 Minuten bei 3-10°C 157,5 g Diketen zutropfen.

Nach 1 Stunde Rührdauer lässt man innert 40 Minuten 25 g Eisessig zutropfen, wobei die Temperatur auf 13°C ansteigt. Sobald die Temperatur wieder fällt, wird das Eisbad entfernt und über Nacht bei Raumtemperatur nachgerührt bis durch Prüfung im Dünnschichtchromatogramm kein Amin mehr feststellbar ist. Hierauf wird abfiltriert, mit Benzin die Mutterlauge ausgewaschen und bei 80°C im Vakuum getrocknet. Man erhält 354 g 4-Acetoacetylamino-acetanilid mit einer Ausbeute von 100 % und einem Reingehalt von 97,0 % (Smp. 145,9°C).

b) Die Umsetzung von 4-Amino-acetanilid mit Diketen wird genau wie unter a) beschrieben wiederholt, mit der einzigen Ausnahme, dass anstelle von Siedegrenzenbenzin Hexan verwendet wird.

Man erhält 4-Acetoacetylaminoacetanilid mit einer Ausbeute von 100 % und einem Reingehalt von 94,2 %.

Beispiel 2

a) In einem 6 l Rührkolben werden 3700 g Siedegrenzenbenzin von 100/125°C, 150 g Essigsäure und 938 g 4-Chlor-2,5-dimethoxyanilin vorgelegt und durch Rühren homogenisiert. Gleichzeitig wird mit einem Kühlbad auf 5-10°C gekühlt und bei dieser Temperatur in ca. 1 Stunde 505 g Diketen zugetropft. Die exotherme Reaktion wird durch ständiges Kühlen kontrolliert, wobei die Temperatur 10°C nicht übersteigen soll. Sobald die Wärmeabgabe beendet ist, wird solange bei Raumtemperatur weitergerührt bis durch Prüfung im Dünnschichtchromatogramm kein Amin mehr feststellbar ist.

Das Reaktionsgemisch wird filtriert, die Mutterlauge mit Siedegrenzenbenzin verdrängt und getrocknet. Man erhält 1358 g 4-Chlor-2,5-dimethoxy-acetoacetylanilid mit einer Ausbeute von 100% und einem Reingehalt von 98,4 %.

b) In einem 1 l Rührkolben werden 300 g Shellsol T (aromatenfreies Gemisch hochverzweigter Paraffinen der Fa. SHELL) mit 80 g 4-Chlor-2,5-dimethoxyanilin homogenisiert und gleichzeitig mit einem Kühlbad auf 5°C gekühlt. Hierauf tropft man 42,8 g Diketen innert 10 Minuten hinzu. Nach 1 Stunde lässt man die Temperatur auf ca. 25°C steigen und rührt weiter bis durch Prüfung im Dünnschichtchromatogramm kein Amin mehr feststellbar ist. Man filtriert und wäscht gut mit Siedegrenzenbenzin von 100/125°C und trocknet. Man erhält so 113,2 g 4-Chlor-2,5-dimethoxy-acetoacetylanilid mit einer Ausbeute von 98 % und einem Reingehalt von 100 % (Smp. 100,1°C).

## B e i s p i e l e 3 - 12

Die in der nachfolgenden Tabelle 1 in Kolonne 2 aufgeführten Amine ergeben bei Diketenisierung nach dem in Beispiel 1a beschriebenen Verfahren die in Kolonne 3 angegebene Ausbeute mit dem in Kolonne 4 angegebenen Reingehalt. In Kolonne 5 ist jeweils der Schmelzpunkt des entsprechenden Acetoacetylarylamids in °C angegeben.

T a b e l l e 1

| Bsp. | Amin | Ausbeute | Reingehalt | Smp |
|---|---|---|---|---|
| 3 | Anilin | 94,8 % | 99,8 % | 84,4° |
| 4 | 4-Methylanilin | 95,5 % | 99,5 % | 94,0° |
| 5 | 2-Chloranilin | 95,7 % | 99,9 % | 106,2° |
| 6 | 3,4-Dichloranilin | 99,6 % | 98,5 % | 88,3° |
| 7 | 3,5-Dimethylanilin | 95,5 % | 99,2 % | 72,9° |
| 8 | 2,4-Dimethylanilin | 97,8 % | 99,2 % | 89,8° |
| 9 | 2-Methoxy-5-chloranilin | 98,5 % | 99,1 % | 92,1° |
| 10 | 2,5-Dichloranilin | 98,0 % | 99,6 % | 94,0° |
| 11 | o-Anisidin | 98,8 % | 97,4 % | 84,9° |
| 12 | O-Toluidin | 97,0 % | 97,4 % | 105,0° |

## B e i s p i e l 13

a) In einem 3 l Rührkolben werden 2000 ml Solvent 30 (16-20 Gew.% Aromaten enthaltendes Gemisch von verzweigten Paraffinen der Schweiz. Spengstoff-Fabrik AG, Dottikon) und 198,2 g 2,5-Diäthoxy-1,4-phenylendiamin homogenisiert. Bei 5-10°C lässt man innert 15 Minuten 202 g Diketen zutropfen. Die leichte exotherme Reaktion wird durch ständiges Kühlen kontrolliert, wobei die Temperatur 10°C nicht übersteigen soll. Nach 1 Stunde Rührdauer tropft man 5 ml 50%-ige Essigsäure ein. Man rührt noch solange weiter, bis durch Prüfung im Dünnschichtchromatogramm kein Amin mehr feststellbar ist und lässt gleichzeitig die Temperatur auf Raumtemperatur ansteigen. Die dünnflüssige, gut rührbare Dispersion wird filtriert und mit Siedegrenzenbenzin 100/125°C gut gewaschen und dann im Vakuum getrocknet. Man erhält 359 g 1,4-Diacetoacetylamino-2,5-diäthoxybenzol mit einer Ausbeute von 98,5 % und einem Reingehalt von 95,4 % (Smp. 206,0°C).

b) Die Umsetzung von 2,5-Diäthoxy-1,4-phenylendiamin mit Diketen wird genau wie unter 13a beschrieben wiederholt, mit der einzigen Ausnahme, dass anstelle von Essigsäure Wasser verwendet wird. Man erhält 1,4-Diacetoacetylamino-2,5-diäthoxy-benzol mit einer Ausbeute von 99 % (361 g) und einem Reingehalt von 99,1 %.

## B e i s p i e l e 14 - 16

Die in der nachfolgenden Tabelle 2 in Kolonne 2 aufgeführten Amine ergeben bei Diketenisierung nach dem in Beispiel 13a beschriebenen Verfahren die in Kolonne 3 angegebene Ausbeute mit dem in Kolonne 4 angegebenen Reingehalt. In Kolonne 5 ist jeweils der Schmelzpunkt des entsprechenden Acetoacetylarylamids in °C angegeben.

Tabelle 2

| Bsp. | | Ausbeute | Reingehalt | Smp. |
|---|---|---|---|---|
| 14 | 2,5-Dimethyl-1,4-phenylen-diamin | 100 % | 96,5 % | >300° |
| 15 | 2,5-Dichlor-1,4-phenylen-diamin | 94,2 % | 97,7 % | 194,1° |
| 16 | 2,5-Dimethoxy-1,4-phenylen-diamin | 97,5 % | 96,5 % | >300° |

Beispiel 17

In einem 5 ℓ-Rührkolben werden 108,2 g p-Phenylendiamin (1,0 Mol) mit 2500 ml Benzin (100/125°C) homogenisiert. Bei 5°C werden innert 15 Min. 219 g Diketen (130% d. Th.) zugegeben; schwach exotherm. Nach 2 Std. Rührdauer tropft man bei gleicher Temperatur 200 ml Essigsäure 60% innert ca. 50 Min. zu (stark exotherm) und rührt bei Raumtemperatur solange weiter, bis nach der Dünnschichtenchromatogramm-Methode kein Amin mehr nachweisbar ist (ca. 20 Std.). Hierauf wird filtriert, mit Benzin gewaschen und hierauf das Lösungsmittel mit Wasser verdrängt. Man erhält 268 g (95,3% d.Th.) 1,4-Diacetoacetylaminobenzol mit einem Reingehalt von 98,2% und einem Schmelzpunkt von 177,6°C.

## Beispiel 18

In 2000 ml Benzin (100/125°C) werden 157 g 2-Methyl-5-chlor-1,4-phenyldendiamin homogenisiert und auf 5°C gekühlt. Im Tropftrichter legt man 20 ml Wasser vor und gibt 218 g Diketen (130% d.Th.) zu. Diese Mischung wird innert 1 Std. bei 5-10°C der Aminsuspension zugegeben. Nach einer weiteren Stunde werden 60 ml Essigsäure bei gleicher Temperatur zugetropft. Dann wird auf 75°C aufgeheizt und bei dieser Temperatur 1 Std. nachgerührt. Hierauf wird filtriert, mit Benzin gedeckt und getrocknet. Man erhält 318 g 2-Methyl-5-chlor-1,4-diacetoacetylaminobenzol (97,9% d.Th.) mit einem Reingehalt von 97,7% und einem Schmelzpunkt von 188,2°C.

## Beispiel 19

In einem Rührkolben werden 329 g einer wasserfeuchten Paste (76,9 %) von 3,3'-Dichlorbenzidin mit 2000 ml Siedegrenzenbenzin von 100/125°C homogenisiert und mit einem Eisbad auf 5°C gekühlt. Bei dieser Temperatur tropft man, bei ständigem Kühlen, um die schwach exotherme Reaktion unter Kontrolle zu halten (Temperatur nicht über 10°C), 218,0 g Diketen innert 20 Minuten zu. Nach 1 Stunde Rührdauer gibt man langsam 120 ml 50%-ige Essigsäure zu. Nach ca. 2 Stunden, wenn die Wärmeabgabe beendet ist, lässt man bei Raumtemperatur weiter rühren, bis durch Prüfung im Dünnschichtchromatogramm kein Amin mehr feststellbar ist. Man filtriert, wäscht mit Siedegrenzenbenzin von 100/125°C, trocknet und erhält 417 g 3,3'-Dichlor-4,4'-diacetoacetylamino-diphenyl mit 99,0 % Ausbeute und einem Reingehalt von 95,3 % (Smp. 211,8°C).

B e i s p i e l 20

Arbeitet man nach der Vorschrift von Beispiel 19 und ersetzt dabei das 3,3'-Dichlorbenzidin mit der entsprechenden Menge 3,3'-Dimethylbenzidin, so erhält man 3,3'-Dimethyl-4,4'-diacetoacetylamino-diphenyl mit einer Ausbeute von 99,5 % (366 g) und einem Reingehalt von 98,5 % (Smp. 202,1°C).

B e i s p i e l 21

In einem 3 l Rührkolben werden 188 g 97,7 %-iges 5-Amino-6-chlorbenzimidazolon mit 1000 ml Siedegrenzenbenzin von 100/125°C homogenisiert. Bei 0 - 5°C lässt man innert 1 Minute 126,5 g Diketen zufliessen (endotherme Reaktion). Nach 1 Stunde Rührdauer entfernt man das Kühlbad und rührt weiter, bis durch Prüfung im Dünnschichtchromatogramm kein Amin mehr feststellbar ist. Die Dispersion wird filtriert, das Nutschgut mit dem Lösungsmittel gewaschen und getrocknet. Man erhält 277 g 5-Acetoacetylamino-6-chlor-benzimidazolon mit 100 % Ausbeute und einem Reingehalt von 93,0 % (Smp. > 300°C).

B e i s p i e l 22

Arbeitet man nach der Vorschrift von Beispiel 21 und ersetzt dabei das 5-Amino-6-chlorbenzimidazolon mit der entsprechenden Menge 5-Amino-benzimidazolon, so erhält man 5-Acetoacetylamino-benzimidazolon mit 95 % Ausbeute (221 g) und einem Reingehalt von 97,0 % (Smp. > 300°C).

Beispiel 23

In einem 350 ml Rührkolben werden 49,5 g 4-Methoxy-3-aminobenzolsulfonsäure-(4'-methoxyphenyl)-ester mit 200 ml Siedegrenzenbenzin von 100/125°C homogenisiert und auf 5°C gekühlt. Nun lässt man 16,2 g Diketen zutropfen. Nach 2 Stunden gibt man 1 ml 50%-ige Essigsäure zu und rührt noch solange weiter, bis durch Prüfung im Dünnschichtchromatogramm kein Amin mehr feststellbar ist. Zur besseren Kristallisation wird dann auf ca. 0°C gekühlt. Dabei kristallisiert das Produkt zu einem beigen Pulver, das filtriert, mit Siedegrenzenbenzin gewaschen und getrocknet wird. Man erhält 62,7 g 4-Methoxy-3-acetoacetylamino-benzolsulfosäure-(4'-methoxyphenyl)-ester mit einer Ausbeute von 99,6% und einem Reingehalt von 99,7% (Smp. 120,4°C).

Beispiele 24 - 26

Die in der nachfolgenden Tabelle 3 in Kolonne 2 aufgeführten Amine ergeben bei Diketenisierung nach dem in Beispiel 23 beschriebenen Verfahren die in Kolonne 3 angegebene Ausbeute mit dem in Kolonne 4 angegebenen Reingehalt. In Kolonne 5 ist jeweils der Schmelzpunkt des entsprechenden Acetoacetylarylamids in Celsiusgraden angegeben.

Tabelle 3

| Bsp. | Amin | Ausbeute | Reingehalt | Smp. |
|---|---|---|---|---|
| 24 | 4-Methoxy-3-amino-benzolsulfosäure-(4'-methylphenyl)-ester | 97,8 % | 98,3 % | 82,3° |
| 25 | 4-Methoxy-3-amino-benzolsulfosäure-(4'-chlorphenyl)-ester | 96,8 % | 99,8 % | 84,2° |
| 26 | 4-Methoxy-3-amino-benzolsulfosäure-(phenyl)-ester | 100 % | 99,0 % | 100,3° |

Patentansprüche

1. Verfahren zur Herstellung von Acetoacetylarylamiden durch Anlagerung von Diketen an primäre carbocyclische oder heterocyclische aromatische Amine in organischen Lösungsmitteln, dadurch gekennzeichnet, dass man das Diketen in die heterogene Suspension des Amins in einem Lösungsmittel oder Lösungsmittelgemisch bestehend aus gesättigten aliphatischen und/oder cycloaliphatischen Kohlenwasserstoffen mit 5 bis 18 C-Atomen zudosiert.

2. Verfahren gemäss Anspruch 1, dadurch gekennzeichnet, dass als Lösungsmittel geradkettige oder verzweigte Paraffine mit 5 bis 18 C-Atome verwendet werden.

3. Verfahren gemäss Anspruch 1, dadurch gekennzeichnet, dass als Lösungsmittel Cycloparaffine mit 5 bis 18 C-Atomen verwendet werden.

4. Verfahren gemäss Anspruch 1, dadurch gekennzeichnet, dass als Lösungsmittel Gemische aus jeweils 5 bis 18 C-Atomen enthaltenden geradkettigen, verzweigten und/oder cyclischen Paraffinen verwendet werden.

5. Verfahren gemäss Anspruch 1, dadurch gekennzeichnet, dass als Lösungsmittel Siedegrenzenbenzine verwendet werden.

6. Verfahren gemäss Anspruch 1, dadurch gekennzeichnet, dass das Diketen bei einer Temperatur zwischen -5 und +15°C zudosiert wird.

0004611

7. Verfahren gemäss Anspruch 1; dadurch gekennzeichnet, dass man von Aminobenzolen ausgeht.

8. Verfahren gemäss Anspruch 1, dadurch gekennzeichnet, dass man von einem Monoaminobenzol der Formel

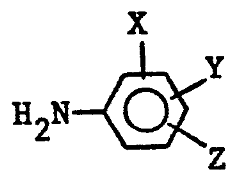

ausgeht, worin X und Y jeweils unabhängig voneinander, Wasserstoff, Halogen, Alkyl oder Alkoxy mit 1-4 C-Atomen , Benzyl und Z Wasserstoff, Chlor, Methyl, Trifluormethyl, Nitro, Cyano, Alkanoylamino mit 1-4 C-Atomen oder eine Gruppe der Formeln

$-CONH_2$, 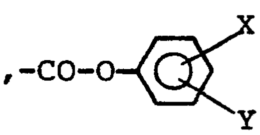 , 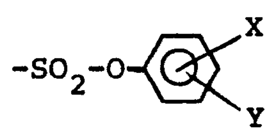 , 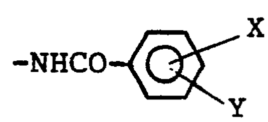

oder -COOR

bedeuten, worin X und Y die oben angegebene Bedeutung haben und R Alkyl mit 1-4 C-Atomen ist.

9. Verfahren gemäss Anspruch 1, dadurch gekennzeichnet, dass man von einem Diaminobenzol der Formel

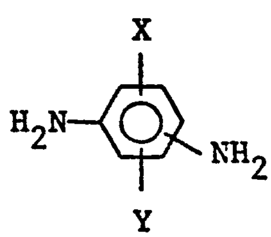

ausgeht, worin X und Y jeweils unabhängig voneinander, Halogen, Alkyl oder Alkoxy mit 1-4 C-Atomen bedeuten.

10. Verfahren gemäss Anspruch 1, dadurch gekennzeichnet, dass man von einem Diaminodiphenyl der Formel

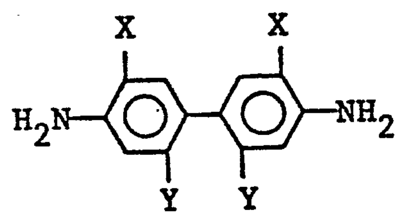

ausgeht, worin X und Y jeweils unabhängig voneinnander Wasserstoff, Halogen, Alkyl oder Alkoxy mit 1-4 C-Atomen bedeuten.

11. Verfahren gemäss Anspruch 1, dadurch gekennzeichnet, dass man von einem heterocyclischen aromatischen Amin der Formel

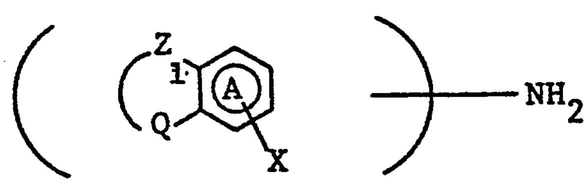

ausgeht, worin X Wasserstoff, Halogen, Alkyl oder Alkoxy mit 1-4 C-Atomen bedeutet, Q eine Gruppe der Formeln -CONH oder -NHCO- und $Z_1$ ein Sauerstoffatom oder eine Gruppe der Formeln $-NR^1-$, -NHCO-, -CONH-, -CH=N-, $-\underset{R^1}{C}=CH-$, oder

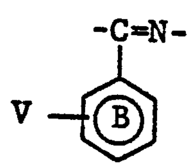

darstellt, wobei $R^1$ Wasserstoff, Alkyl mit 1-4 C-Atomen oder gegebenenfalls durch Halogen, Alkyl oder Alkoxy mit 1-4 C-Atomen substituiertes Phenyl und V Wasserstoff, Halogen, Alkyl oder Alkoxy mit 1-4 C-Atomen bedeuten und die Aminogruppe an die Phenylreste A oder B gebunden sein kann.

12. Verfahren gemäss Anspruch 11, dadurch gekennzeichnet, dass man von einem heterocyclischen Amin der Formel

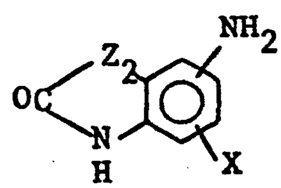

ausgeht, worin X die in Anspruch 11 angegebene Bedeutung hat und $Z_2$ ein Sauerstoffatom oder die Gruppe -NH- bedeutet.

0004611

Nummer der Anmeldung

EP 79 10 0881

Europäisches Patentamt

# EUROPÄISCHER RECHERCHENBERICHT

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | DE - A - 1 931 213 (FMC CORP.)<br>* Seite 6; Beispiel 1 *<br>---- | 1-12 | C 07 C 102/00<br>143/675<br>C 07 D 235/26 |
| | | | RECHERCHIERTE SACHGEBIETE (Int. Cl.²) |
| | | | C 07 C 102/00<br>103/34 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 28-06-1979 | PAUWELS |

EPA form 1503.1 06.78